(19) Europäisches Patentamt / European Patent Office / Office européen des brevets

(11) **EP 1 699 503 B1**

(12) **EUROPEAN PATENT SPECIFICATION**

(45) Date of publication and mention
of the grant of the patent:
**14.03.2012 Bulletin 2012/11**

(21) Application number: **04804273.3**

(22) Date of filing: **23.12.2004**

(51) Int Cl.:
*A61L 31/10* (2006.01)     *A61L 29/08* (2006.01)
*A61L 31/16* (2006.01)     *A61L 29/16* (2006.01)

(86) International application number:
**PCT/EP2004/014682**

(87) International publication number:
**WO 2005/063319 (14.07.2005 Gazette 2005/28)**

(54) **Devices coated with PEC polymers**

Vorrichtungen beschichtigt mit PEK-Polymere

Dispositfs revêtus de polymères de type PEC

(84) Designated Contracting States:
**AT BE BG CH CY CZ DE DK EE ES FI FR GB GR
HU IE IS IT LI LT LU MC NL PL PT RO SE SI SK TR**

(30) Priority: **24.12.2003 GB 330031
07.05.2004 GB 410261**

(43) Date of publication of application:
**13.09.2006 Bulletin 2006/37**

(73) Proprietors:
• **Novartis AG**
**4056 Basel (CH)**
Designated Contracting States:
**BE BG CH CY CZ DE DK EE ES FI FR GB GR HU
IE IS IT LI LT LU MC**
• **Novartis Pharma GmbH**
**1230 Wien (AT)**
Designated Contracting States:
**AT**

(72) Inventors:
• **AUSBORN, Michael**
**79540 Lörrach (DE)**
• **KISSEL, Thomas**
**79219 Grunern (DE)**

(74) Representative: **Bohmann, Christine**
**Novartis AG**
**4002 Basel (CH)**

(56) References cited:
**WO-A-95/06077     WO-A-03/037397
US-A- 5 376 120**

• **DADSETAN M ET AL: "In vivo biocompatibility
and biodegradation of poly(ethylene carbonate)"
JOURNAL OF CONTROLLED RELEASE,
ELSEVIER SCIENCE PUBLISHERS B.V.
AMSTERDAM, NL, vol. 93, no. 3, 12 December
2003 (2003-12-12), pages 259-270, XP004476715
ISSN: 0168-3659**

**Description**

The present invention relates to a device implantable into a human or animal body comprising a biodegradable polymer as well as the use of such device for the controlled release of a pharmacologically active agent.

Many humans suffer from circulatory diseases caused by a progressive blockage of the blood vessels that perfuse the heart and other major organs. Severe blockage of blood vessels in such humans often leads to ischemic injury, hypertension, stroke or myocardial infarction. Atherosclerotic lesions which limit or obstruct coronary or periphery blood flow are the major cause of ischemic disease related morbidity and mortality including coronary heart disease and stroke. To stop the disease process and prevent the more advanced disease states in which the cardiac muscle or other organs are compromised, medical revascularization procedures such as percutaneous transluminal coronary angioplasty (PCTA), percutaneous transluminal angioplasty (PTA), atherectomy, bypass grafting or other types of vascular grafting procedures are used.

Re-narrowing (e.g. restenosis) of an artherosclerotic coronary artery after various revascularization procedures occurs in 10-80% of patients undergoing this treatment, depending on the procedure used and the aterial site. Besides opening an artery obstructed by atherosclerosis, revascularization also injures endothelial cells and smooth muscle cells within the vessel wall, thus initiating a thrombotic and inflammatory response. Cell derived growth factors such as platelet derived growth factor, infiltrating macrophages, leukocytes or the smooth muscle cells themselves provoke proliferative and migratory responses in the smooth muscle cells. Simultaneous with local proliferation and migration, inflammatory cells also invade the site of vascular injury and may migrate to the deeper layers of the vessel wall. Proliferation/migration usually begins within one to two days post-injury and, depending on the revascularization procedure used, continues for days and weeks.

Both cells within the atherosclerotic lesion and those within the media migrate, proliferate and/or secrete significant amounts of extracellular matrix proteins. Proliferation, migration and extracellular matrix synthesis continue until the damaged endothelial layer is repaired at which time proliferation slows within the intima. The newly formed tissue is called neointima, intimal thickening or restenotic lesion and usually results in narrowing of the vessel lumen. Further lumen narrowing may take place due to constructive remodeling, e.g. vascular remodeling, leading to further intimal thickening or hyperplasia.

There are also atherosclerotic lesions which do not limit or obstruct vessel blood flow but which form the so-called "vulnerable plaques". Such atherosclerotic lesions or vulnerable plaques are prone to rupture or ulcerate, which results in thrombosis and thus produces unstable angina pectoris, myocardial infarction or sudden death. Inflamed atherosclerotic plaques can be detected by thermography.

Complications associated with vascular access devices is a major cause of morbidity, for example in hemodialysis patients, e.g. caused by outflow stenoses in the venous circulation. Venous neointimal hyperplasia characterized by stenosis and subsequent thrombosis accounts for the overwhelming majority of pathology resulting in dialysis graft failure. Vascular access related morbidity was found to account for about 23 percent of all hospital stays for advanced renal disease patients and to contribute to as much as half of all hospitalization costs for such patients. Additionally, vascular access dysfunction in chemotherapy patients is generally caused by outflow stenoses in the venous circulation and results in a decreased ability to administer medications to cancer patients. Often the outflow stenoses is so severe as to require intervention. Additionally, vascular access dysfunction in total parenteral nutrition (TPN) patients is generally caused by outflow stenoses in the venous circulation and results in reduced ability to care for these patients.

Up to the present time, there has not been any effective treatment for the prevention or reduction of vascular access dysfunction that accompany the insertion or repair of an indwelling shunt, fistula or catheter, such as a large bore catheter, into a vein in a mammal, particularly a human patient.

Stents have been found to be useful instead of or along with angioplasty to reduce the renarrowing of an artery that occurs after balloon angioplasty or other procedures that use catheters. Stents help restore normal blood flow and keep an artery open after the intervention with the balloon catheter, however, restenosis (reclosure) is also a problem with the stent procedure. Reocclusion following stenting may be due to both restenotic lesion formation within the stent boundaries and constrictive remodeling at both the proximal and distal margins of the local delivery device or system, e.g. stent.

Recently stents have been proposed which are coated with drugs that are slowly released and help keep the vessel from reclosing. However, major obstacles associated with drug-coated stents are the biodegradability of the polymer in which the drug may be incorporated and the biocompatibility of the surfaces of the medical devices. Further important for the long-term success of the procedure are the mechanical properties of the polymer.

Accordingly, there continues to exist a need for effective treatment and drug delivery systems for revascularization procedure, e.g. for preventing or treating intimal thickening or restenosis that occurs after injury, e.g. vascular injury, including e.g. surgical injury, e.g. revascularization-induced injury, e.g. also in heart or other grafts, for a stabilization procedure of vulnerable plaques, or for the prevention or treatment of vascular access dysfunctions.

In accordance with the present invention it has now surprisingly been found that a superior medical device implantable into a human or animal body may be obtained by coating the device with a biodegradable polymer which comprises ethylene carbonate units of the formula -(-C(O)-O-CH$_2$-CH$_2$-O-)- having an ethylene carbonate content of 70 to 100 Mol%, an intrinsic viscosity of 0.4 to 4.0 dl/g measured in chloroform at 20°C at a concentration of 1 g/dl and a glass transition temperature of from 5 to 50°C, e.g. 15 to 50°C, degradable by surface erosion which is governed by a non-hydrolytic mechanism. The polymer used to coat the device of the invention is hereinafter referred to as the polymer of the invention. It shows superior biocompatibility, biodegradability, and mechanical properties, e.g. hard-elastic properties, e.g. viscoelasticity, as well as superior release characteristics of a pharmacologically active agent incorporated, e.g. dissolved, dispersed or suspended, in the polymer. According to the invention it has been found that this unique combination of properties can be exploited to improve the long-term success of procedures, e.g. stenting or other grafting procedures, as hereinabove decribed.

As used herein the meaning of the terms "polymer of the invention", "polymeric matrix of the invention", "polymer used according to the invention", "poly (ethylene carbonate) (PEC)", "(co)-polymer" or in some cases "(co)-polymer used in the invention" or "(co)-polymer used in the device of the invention" is to be understood as equivalent.

By "biocompatible" is meant a material which elicits no or minimal negative tissue reaction including e.g. thrombus formation and/or inflammation.

In accordance with the particular finding of the present invention, there is provided a device according to claim 1 comprising a biodegradable polymer which comprises ethylene carbonate units of the formula A

$$-(-C(O)-O-CH_2CH_2-O-)-  \qquad A$$

having an ethylene carbonate content of 70 to 100 Mol%, an intrinsic viscosity of 0.4 to 4.0 dl/g measured in chloroform at 20°C at a concentration of 1 g/dl and a glass transition temperature of from 5 to 50°C, e.g. 15 to 50°C, degradable by surface erosion which is governed by a non-hydrolytic mechanism, hereinafter referred to as device of the invention.

In a further embodiment of the invention there is provided a use of a biodegradable polymer which comprises ethylene carbonate units of the formula A

$$-(-C(O)-O-CH_2-CH_2-O-)-  \qquad A$$

having an ethylene carbonate content of 70 to 100 Mol%, an intrinsic viscosity of 0.4 to 4.0 dl/g measured in chloroform at 20°C at a concentration of 1 g/dl and a glass transition temperature of from 5 to 50°C, e.g. 15 to 50°C, degradable by surface erosion which is governed by a non-hydrolytic mechanism, for the coating of a device, e.g. a medical device implantable into a human or animal body, hereinafter referred to as use of the invention.

The medical device may be chosen from catheters, guide wires, balloons, filters, vascular grafts, graft connectors, tubing, implants, sutures, surgical staples, stentgrafts and stents. Preferably, the medical device is a stent.

The stent according to the invention can be any stent, including self-expanding stent, or a stent that is radially expandable by inflating a balloon or expanded by an expansion member, or a stent that is expanded by the use of radio frequency which provides heat to cause the stent to change its size.

Stents may be commonly used as a tubular structure left inside the lumen of a duct or vessel to relieve an obstruction. They may be inserted into the duct lumen in a non-expanded form and are then expanded autonomously (self-expanding stents) or with the aid of a second device in situ, e.g. a catheter-mounted angioplasty balloon which is inflated within the stenosed vessel or body passageway in order to disrupt the obstructions associated with the wall components of the vessel and to obtain an enlarged lumen. Alternatively, stents being easily deformed at lower temperature to be inserted in the hollow tubes may be used: after deployment at site, such stents recover their original shape and exert a retentive and gentle force on the internal wall of the hollow tubes, e.g. of the esophagus or trachea.

Any commercially available stent may be used, e.g. JOSTENT® Flex, JOMED, JOSTENT® SelfX, JOSTENT® Peripheral, JOSTENT® Renal, Biodivysion™ (Biocompatibles Ltd., UK), BX high velocity Stainless Steel L316™ (Cordis, Johnson & Johnson Co., USA), NIR Primo Stainless Steel 316L™, NIRoyal Stainless Steel 316L™ (coated with a 7 μm layer of gold-plating), Radius self-expanding Nitinol™ stent (Medinol, Scimed, Boston Scientific Co., USA), S6™ and S7™ (AVE, Metronic, USA), Multilink Duett™ and Ultra™ (ACS, Guidant S.A., Belgium).

The exterior surface of the device may consist of metal, e.g. gold, silver, platinum, stainless steel, nickel, titanium and biocompatible alloys thereof, or a biodegradable and/or biocompatible organic or inorganic polymer, e.g. fibrin, polytetrafluoroethylene (PTFE), poly-p-xylylene (PPX), silicone, silicone rubber, nylon and/or polyethylene perthalate (Dacron), or of metal pre-covered with one or more biodegradable and/or biocompatible organic or inorganic polymer, e.g. pre-covered with PPX.

The polymer used in the device of the invention and its process of manufacture are disclosed in WO 95/06077. .

The ethylene carbonate content of the polymer used according to the invention is from 70 to 100 Mol%, particularly 80-100%, preferably from 90-99.9%, such as from 94 to 99.9%. The intrinsic viscosity of the (co)-polymer is from 0.4 to 4.0 dl/g, measured in chloroform at 20°C and a concentration of 1 g/dl in chloroform of 0.4 to 3.0 dl/g. Its glass transition temperature is from 5 to 50°C, e.g. 15° or 18° to 50°C.

As a consequence of their production method the (co)-polymers contain in most cases as a co-unit the ethylene oxide unit of the formula B

$$-(-CH_2-CH_2-O-)- \qquad\qquad B$$

In the (co)-polymers used in the invention, if exposed to an aqueous medium, e.g. a phosphate-buffered saline of pH 7.4 practically no medium will be transported to their bulk part. Therefore no bulk erosion will occur and the remaining polymer mass will be kept constant (100%). Its embedded drug, if sensitive to moisture, remains stable.

The (co)-polymer used in the invention is produced by copolymerization of ethylene oxide and $CO_2$ in a molar ratio of from 1:4 to 1:5 under the influence of a catalyst. In the scope of this reaction the introduction of ethylene oxide units in the (co)-polymer chain is possible, if two epoxide molecules react with each other without intervention of a $CO_2$ molecule, i.e. if an oxy anion intermediate attacks another ethylene oxide molecule before being carboxylated by $CO_2$. It is thus probable that the (co)-polymer contains several ethylene oxide units.

The (co)-polymer used in the invention, if containing ethylene oxide units, has a random distribution of ethylene carbonate and ethylene oxide units according to the sum formula

$$A_m\text{-}B_n = -(C(O)\text{-}O\text{-}CH_2\text{-}CH_2\text{-}O\text{-}_m\text{-}(-CH_2\text{-}CH_2\text{-}O-)\text{-}_n$$

in which

$$\frac{m}{n+m} \times 100 = 70 \text{ to } 100.$$

In the process the ethylene oxide unit content and thus the content of ether functions, which delays or inhibits the biodegradation speed of the (co)-polymer, is reduced considerably by specifying the reaction conditions such as the described molar ratio's of the reaction components, the reaction temperature and further by choosing an appropriate catalyst, e.g. such prepared from Zn $(C_2H_5)_2$ and water or acetone or a di- or a triphenol, e.g. phloroglucin, in a molar ratio of from 0.9:1 to 1:0.9 or 2:1 to 1:2 respectively, or preferably prepared from Zn $(C_2H_5)_2$ and a diol, especially ethylene glycol, in a molar ratio of from 0.9:1 to 1:0.9.

The process is preferably carried out in a solvent or dispersing agent system of an organic solvent, e.g. dioxane and $CO_2$. $CO_2$ is preferably applied in liquid form and is present in an excess. The pressure is preferably from 20 to 70 bar and the temperature preferably from 10 to 80°C, especially from 20 to 70°C.

The polymers thus obtained comprise usually less than 15% of ether functions, preferably less than 10%, particularly less than 5%, e.g. less than 3%. The poly(ethylenecarbonate)s, if prepared using the catalyst from ethylene glycol or acetone and diethylzinc exhibit low polydispersities (Mw/Mn), usually less than 5, such as less than 2.5.

According to the above process the catalyst or a part of it is considered to be the chain initiator for the (co)-polymerisation. When the reaction is finished and the chain is complete, its final terminal group is a hydroxyl group. The opposite site of the chain, there where the chain was started up, may be occupied by the catalyst group or a fragment of it. If the catalyst is prepared from ethylene glycol and diethylzinc or water and diethylzinc, both ends of a polymer chain are supposed to be identical. However, if the catalyst is prepared from a di-or triphenol and diethylzinc, the aromatic group will be incorporated into the end of a chain, where the chain starts up, whereas the other end of the chain will be a hydroxyl group. It was shown that poly(ethylene carbonate), if one of its terminal groups is blocked, e.g. by an aromatic initiator such as phloroglucin, is slower biodegradable. Alternatively, a later derivatization of a terminal hydroxyl group may also be considered, e.g. by esterification, to block terminal hydroxyl groups and to control the biodegradation of the poly (ethylene carbonate)s used in the invention. Suitable terminal ester groups are biocompatible ester groups, like $(C_{1\text{-}48})$ fatty acid ester groups, preferably $(C_{1\text{-}30})$, especially $(C_{1\text{-}18})$ fatty acid ester groups, e.g. the ester groups of acetic acid and stearic acid, or a carbonic acid ester group, e.g. the ethylene carbonate group, or the pamoic ester group or a lactic or glycolic or polylactic or polyglycolic or polylactic-co-glycolic acid ester group.

The poly (ethylene carbonate)s used in the invention are stable for several hours in hot distilled or demineralized water (90-100°C). A significant increase of the glass transition temperature is observed after exposure to boiling bidistilled water during 5 hours, e.g. up to above 18°C, e.g. 28°C. By performing this purification step, a higher polymer purity and better processable polymer is attained.

The poly (ethylene carbonate) portion of the (co)-polymers used in the invention is not hydrolysable, i.e. during at least 1 month by hydrolytic enzymes under physiological conditions or by water at pH 12 and 37°C.

However, it has been found that the (co)-polymers used in the invention degrade in vivo and in vitro by surface erosion under the influence of the superoxide radical anion $O_2^{\cdot-}$. Superoxide radical anions $O_2^{\cdot-}$ are generated in inflammatory cells such as those which may occur during the restenosis process. Accordingly, when a drug is incorporated into the polymer of the invention, the rate of drug release may increase in case of restenosis processes and may slow down in case of reduced restenosis rates. The polymer of the invention may serve as an "on-demand" drug-eluting coating, e.g. matrix, which releases an incorporated drug at an inflamed implantation site, e.g. by contact with macrophages.

The degradation rate of the (co)-polymers of the invention may be adjusted within wide limits, depending on their molecular weight, their ethylene oxide content, the identity of the terminal groups, e.g. biocompatible ester groups, and the presence of $O_2^{\cdot-}$ -radical scavengers, e.g. vitamin C, and may last between 5 days and 6 months or longer, e.g. up to 1 year. A radical scavenger may be embedded in the (co)-polymer as an additive.

As the polymer used in the invention degrades by surface erosion, the overall polymer mass degradation may be adjusted by the amount of polymer implanted and the specific surface to volume ratio of the implant.

The molecular weight (Mw) of the (co)-polymers of the invention is from 80,000, preferably from 100,000, particularly from 200,000 to 2,000,000 Daltons, determined by gel permeation chromatography with methylene chloride as the eluant and polystyrene as the reference.

The (co)-polymer used in the device of the invention may be used alone or in combination with another polymer suitable to coat a medical device, e.g. a stent, implantable into a human or animal body. Suitable polymers for use in combination with the polymer used in the device of the invention may be one or more of the following: hydrophilic, hydrophobic or biocompatible biodegradable materials, e.g. polycarboxylic acids; cellulosic polymers; starch; collagen; hyaluronic acid; gelatin; lactone-based polyesters or copolyesters, e.g. polylactide; polyglycolide; polylactide-glycolide; polycaprolactone; polycaprolactone-glycolide; poly(hydroxybutyrate); poly(hydroxyvalerate); polyhydroxy(butyrate-co-valerate); polyglycolide-co-trimethylene carbonate; poly(diaxanone); polyorthoesters; polyanhydrides; polyaminoacids; polysaccharides; polyphospoeters; polyphosphoester-urethane; polycyanoacrylates; polyphosphazenes; poly(ether-ester) copolymers, e.g. PEO-PLLA, fibrin; fibrinogen; or mixtures thereof; and biocompatible non-degrading materials, e.g. poly-p-xylylene (PPX), polyurethane; polyolefins; polyesters; polyamides; polycaprolactame; polyimide; polyvinyl chloride; polyvinyl methyl ether; polyvinyl alcohol or vinyl alcohol/olefin copolymers, e.g. vinyl alcohol/ethylene copolymers; polyacrylonitrile; polystyrene copolymers of vinyl monomers with olefins, e.g. styrene acrylonitrile copolymers, ethylene methyl methacrylate copolymers; polydimethylsiloxane; poly(ethylene-vinylacetate); acrylate based polymers or coplymers, e.g. polybutylmethacrylate, poly(hydroxyethyl methylmethacrylate); polyvinyl pyrrolidinone; fluorinated polymers such as polytetrafluoethylene; cellulose esters e.g. cellulose acetate, cellulose nitrate or cellulose propionate. For example, in one aspect of the invention, the metal stent may be pre-covered with a biocompatible, non-biodegradable polymer, e.g. PPX, and then covered with the poly (ethylene carbonate) polymer of the invention.

The (co)-polymers used in the invention are advantageously combined with pharmacologically active agents. For example, the pharmacologically active agents may be incorporated into the polymeric matrix. Since under in vitro and in vivo conditions no bulk erosion occurs and the active compound is protected by the polymer, the active compound is released as soon as it appears at the matrix surface due to surface erosion of the matrix. Advantageously, the size of the pharmacologically active compound molecule does not influence its release rate. However, according to the invention it has been found that different particle sizes may for example be used to influence the release rate to a certain extent.

In a series of further specific or alternative embodiments the invention also provides a device as hereinabove described further comprising a pharmacologically active agent incorporated, e.g. dissolved, dispersed or suspended, in the polymer.

In one aspect the invention provides the use of the device of the invention for the controlled, e.g. sustained, delivery of the pharmacologically active agent, e.g. of sufficient pharmacological activity, at or near the coated surfaces of the device.

In yet a further aspect the present invention provides a device as hereinabove described in form of a drug-eluting stent.

The term "sustained release" or "controlled release" as used herein, means that the (co)-polymer used releases no more than 10, 20, 30, 40 or 50% to 60, 70, 80, or 90% by weight of the pharmacologically active agent dissolved or dispersed therein within 3 to 10, e.g. 7, days after implantation of the device into a human or animal body.

In yet a further aspect the invention provides the use of the (co)-polymer as defined herein as a matrix for the controlled release of a pharmaceutically active agent from a device, e.g. a medical device implantable into a human or animal body, e.g. stent.

As used herein, the term "pharmacologically active agent" comprises any substances which may yield a physiological response when administered to a living organism. Such substance should be administered in a "therapeutically effective amount".

As used herein, the term "therapeutically effective amount" refers to an amount or concentration which is effective in reducing, eliminating, treating, preventing or controlling the symptoms of a disease or condition affecting a mammal. The term "controlling" is intended to refer to all processes wherein there may be a slowing, interrupting, arresting or stopping of the progression of the diseases and conditions affecting the mammal. However, "controlling" does not necessarily indicate a total elimination of all disease and condition symptoms, and is intended to include prophylactic treatment.

The appropriate therapeutically effective amount is known to one of ordinary skill in the art as the amount varies with the therapeutic compound being used and the indication which is being addressed.

As used herein the meaning of the terms "pharmaceutical active agent", "active ingredient", "pharmacologically active compound", "active substance" or "drug substance" is to be understood as equivalent.

According to the invention, the pharmacologically active agent may be chosen from at least one of:

a) an immunosuppressive agent, e.g. a calcineurin inhibitor, e.g. a cyclosporin, for example cyclosporin A, ISA tx 247 or FK506,

b) an EDG-receptor agonist having lymphocyte depleting properties, e.g. FTY720 (2-amino-2-[2-(4-octylphenyl) ethyl]propane-1,3-diol in free form or in a pharmaceutically acceptable salt form, e.g. the hydrochloride) or an analogue such as described in WO96/06068 or WO 98/45249, e.g. 2-amino-2-{2-[4-(1-oxo-5-phenylpentyl)phenyl] ethyl}propane-1,3-diol or 2-amino-4-(4-heptyloxyphenyl)-2-methylbutanol in free form or in a pharmaceutically acceptable salt form,

c) an anti-inflammatory agent, e.g. a steroid, e.g. a corticosteroid, e.g. dexamethasone or prednisone, a NSAID, e.g. a cyclooxygenase inhibitor, e.g. a cox-2 inhibitor, e.g. celecoxib, rofecoxib, etoricoxib or valdecoxib, an ascomycin, e.g. ASM981 (or pimecrolimus), a cytokine inhibitor, e.g. a lymphokine inhibitor, e.g. an IL-1, -2 or -6 inhibitor, for example pralnacasan or anakinra, or a TNF inhibitor, for instance Etanercept, or a chemokine inhibitor;

d) an anti- thrombotic or anti-coagulant agent, e.g. heparin or a glycoprotein IIb/IIIa inhibitor, e.g. abciximab, eptifibatide or tirofibran;

e) an antiproliferative agent, e.g.

a microtubule stabilizing or destabilizing agent including but not limited to taxanes, e.g. taxol, paclitaxel or docetaxel, vinca alkaloids, e.g. vinblastine, especially vinblastine sulfate, vincristine especially vincristine sulfate, and vinorelbine, discodermolides or epothilones or a derivative thereof, e.g. epothilone B or a derivative thereof;

a protein tyrosine kinase inhibitor, e.g. protein kinase C or PI(3) kinase inhibitor, for example staurosporin and related small molecules, e.g. UCN-01, BAY 43-9006, Bryostatin 1, Perifosine, Limofosine, midostaurin, CGP52421, RO318220, RO320432, GO 6976, Isis 3521, LY333531, LY379196, SU5416, SU6668, AG1296, etc. Midostaurin is a derivative of the naturally occurring alkaloid staurosporine with the chemical name (*N*-[(9*S*,10*R*,11*R*,13*R*)-2,3,10,11,12,13-hexahydro-10-methoxy-9-methyl-1-oxo-9,13-epoxy-1*H*,9*H*-diindolo[1,2,3-gh:3',2',1'-lm]pyrrolo [3,4-j][1,7]benzodiazonin-11-yl]-*N*-methylbenzamide), and has been specifically described in the European patent No. 0 296 110, as well as in US patent No. 5;093,330, and Japanese Patent No. 2 708 047. Midostaurin was originally identified as an inhibitor of protein kinase C (PKC) (Meyer T, Regenass U, Fabbro D, et al: Int J Cancer 43: 851-856, 1989).

a compound or antibody which inhibits the PDGF receptor tyrosine kinase or a compound which binds to PDGF or reduces expression of the PDGF receptor e.g. a N-phenyl-2-pyrimidine-amine derivative, CT52923, RP-1776, GFB-111, a pyrrolo[3,4-c]-beta-carboline-dione, etc.;

a compound or antibody which inhibits the EGF receptor tyrosine kinase or a compound which binds to EGF or reduces expression of the EGF receptor e.g. EGF receptor, ErbB2, ErbB3 and ErbB4 or bind to EGF or EGF related ligands, and are in particular those compounds, proteins or monoclonal antibodies generically and specifically disclosed in WO 97/02266, e.g. the compound of ex. 39, or in EP O 564 409, WO 99/03854, EP 0520722, EP 0 566 226, EP 0 787 722, EP 0 837 063, US 5,747,498, WO 98/10767, WO 97/30034, WO 97/49688, WO 97/38983 and, especially, WO 96/30347 (e.g. compound known as CP 358774), WO 96/33980 (e.g. compound ZD 1839, Iressa) and WO 95/03283 (e.g. compound ZM105180); e.g. trastuzumab (Herpetin[R]), cetuximab, OSI-774, CI-1033, EKB-569, GW-2016, E1.1, E2.4, E2.5, E6.2, E6.4, E2.11, E6.3 or E7.6.3, retinoic acid, alpha-, gamma- or delta-tocopherol or alpha-, gamma- or delta-tocotrienol, or compounds affecting GRB2, IMC-C225; or

a compound or antibody which inhibits the VEGF receptor tyrosine kinase or a VEGF receptor or a compound which binds to VEGF, e.g. proteins, small molecules or monoclonal antibodies generically and specifically disclosed in WO 98/35958, e.g. 1-(4-chloroanilino)-4-(4-pyridylmethyl)phthalazine or a pharmaceutically acceptable salt thereof, e.g. the succinate, or in WO 00/09495, WO 00/27820, WO 00/59509, WO 98/11223, WO 00/27819, WO 00/37502, WO 94/10202 and EP 0 769 947, those as described by M. Prewett et al in Cancer Research 59 (1999) 5209-5218, by F. Yuan et al in Proc. Natl. Acad. Sci. USA, vol. 93, pp. 14765-14770, Dec. 1996, by Z. Zhu et al in Cancer Res. 58, 1998, 3209-3214, by J. Mordenti et al in Toxicologic Pathology, Vol. 27, no. 1, pp 14-21, 1999, Angiostatin™, described by M. S. O'Reilly et al, Cell 79, 1994, 315-328, Endostatin™, described by M. S. O'Reilly et al, Cell 88, 1997, 277-285, anthranilic acid amides, ZD4190; ZD6474, SU5416, SU6668 or anti-VEGF antibodies or anti-VEGF receptor antibodies, e.g. RhuMab;

f) a statin, e.g. having HMG-CoA reductase inhibition activity, e.g. fluvastatin, lovastatin, simvastatin, pravastatin, atorvastatin, cerivastatin, pitavastatin, rosuvastatin or nivastatin;

g) a compound, protein, growth factor or compound stimulating growth factor production that will enhance endothelial regrowth of the luminal endothelium, e.g. FGF, IGF;

h) a matrix metalloproteinase inhibitor, e.g. batimistat, marimistat, trocade, CGS 27023, RS 130830 or AG3340;

k) a modulator (i.e. antagonists or agonists) of kinases, e.g. JNK, ERK1/2, MAPK or STAT;

l) a compound stimulating the release of (NO) or a NO donor, e.g. diazeniumdiolates, S-nitrosothiols, mesoionic oxatriazoles, isosorbide or a combination thereof, e.g. mononitrate and/or dinitrate;

m) a somatostatin analogue, e.g. octreotide, lanreotide, vapreotide or a cyclohexapeptide having somatostatin agonist properties, e.g. cyclo[4-($NH_2$-$C_2H_4$-NH-CO-O)Pro-Phg-DTrp-Lys-Tyr(Bzl)-Phe]; or a modified GH analogue chemically linked to PEG, e.g. Pegvisomant;

n) an aldosterone synthetase inhibitor or aldosterone receptor blocker, e.g. eplerenone, or a compound inhibiting the renin-angiotensin system, e.g. a renin inhibitor, e.g. SPP100, an ACE inhibitor, e.g. captopril, enalapril, lisinopril, fosinopril, benazepril, quinapril, ramipril, imidapril, perindopril erbumine, trandolapril or moexipril, or an ACE receptor blocker; e.g. losartan, irbesartan, candesartan cilexetil, valsartan or olmesartan medoxomil;

o) mycophenolic acid or a salt thereof, e.g. sodium mycophenolate, or a prodrug thereof, e.g. mycophenolate mofetil;

p) a rapamycin derivative. Rapamycin is a known macrolide antibiotic produced by Streptomyces hygroscopicus, which inhibits mTOR. By rapamycin derivative having mTOR inhibiting properties is meant a substituted rapamycin, e.g. a 40-substituted-rapamycin or a 16-substituted rapamycin, or a 32-hydrogenated rapamycin. Representative rapamycin derivatives are e.g. 32-deoxorapamycin, 16-pent-2-ynyloxy-32-deoxorapamycin, 16-pent-2-ynyloxy-32 (S or R)-dihydro-rapamycin, 16-pent-2-ynyloxy-32(S or R)-dihydro-40-O-(2-hydroxyethyl)-rapamycin, 40-[3-hydroxy-2-(hydroxymethyl)-2-methylpropanoate]-rapamycin (also called CCI779) or 40-epi-(tetrazolyl)-rapamycin (also called ABT578). A preferred compound is e.g. 40-0-(2-hydroxyethyl)-rapamycin disclosed in Example 8 in WO 94/09010, or 32-deoxorapamycin or 16-pent-2-ynyloxy-32(S)-dihydro-rapamycin as disclosed in WO 96/41807. Rapamycin derivatives may also include the so-called rapalogs, e.g. as disclosed in WO 98/02441 and WO01/14387, e.g. AP23573;

q) an antibiotic.

The above list further comprises the pharmaceutically acceptable salts, the corresponding racemates, diastereoisomers, enantiomers, tautomers as well as the corresponding crystal modifications of above disclosed compounds where present, e.g. solvates, hydrates and polymorphs. Further comprised are metabolites and drug-conjugates.

By antibody is meant monoclonal antibodies, polyclonal antibodies, multispecific antibodies formed from at least 2 intact antibodies, and antibodies fragments so long as they exhibit the desired biological activity.

The preferred pharmacologically active agents according to the invention are chosen from at least one of a rapamycin derivative having mTOR inhibiting properties or rapamycin, an EDG-receptor agonist having lymphocyte depleting properties, a cox-2 inhibitor, pimecrolimus, a cytokine inhibitor, a chemokine inhibitor, an antiproliferative agent, a statin, a protein, growth factor or compound stimulating growth factor production that will enhance endothelial regrowth of the luminal endothelium, a matrix metalloproteinase inhibitor, a somatostatin analogue, an aldosterone synthetase inhibitor or aldosterone receptor blocker and a compound inhibiting the renin-angiotensin system. Most preferably pharmacologically active agents selected from a calcineurin inhibitor, mycophenolic acid, rapamycin and midostaurin or a salt thereof or prodrug thereof, may be used.

Any of the above listed compounds, alone or in combination, or any other compound useful in the treatment or prevention of neointimal proliferation and thickening, restenosis and/or vascular occlusion following vascular injury, vascular access dysfunction or for promoting tissue healing may be used for incorporation into the polymer used in the device of the invention.

In a further aspect the invention provides a device coated with a (co)-polymer as defined herein comprising a pharmacologically active agent as described above, showing non-hydrolytic surface erosion, especially with a linear, especially a 1:1 linear correlation of active compound release and non-hydrolytic (co)-polymer mass degradation and active compound protection in the (co)-polymer matrix.

The composition comprising the (co)-polymer and the pharmacologically active agent may further comprise pharmaceutically acceptable excipients, e.g. ionic or non-ionic surfactants, adhesives, stabilizers, antioxidants, lubricants and/or pH regulators. It will be appreciated that such further ingredients are well known in the art.

The pharmacologically active agent may be present in a concentration of from 0.01 to 99 % by weight (wt%). The typical dosage of the pharmacologically active agent varies within a wide range and depends on various factors, such as the particular requirements of each receiving individual, the used active agent, the circumstance under which it is applied, and the particular medical device used. The dosage is generally within the range of 0.001-100 mg/kg, e.g. 0.001-10 mg/kg, body weights, however, certain circumstances may require other ranges.

The local delivery according to the present invention allows for high concentration of the drug(s) at the disease site with low concentration of circulating compound. The amount of drug(s) used for local delivery applications will vary depending on the compounds used, the condition to be treated and the desired effect. For purposes of the invention, a therapeutically effective amount will be administered; for example, the drug delivery device or system is configured to release the active agent and/or the active co-agent at a rate of 0.001 to 800 $\mu$g/day, preferably 0.001 to 200 $\mu$g/day. By therapeutically effective amount is intended an amount sufficient to inhibit cellular proliferation and resulting in the prevention and treatment of the disease state. Specifically, for the prevention or treatment of vascular problems e.g. after revascularization, or antitumor treatment, local delivery may require less compound than systemic administration.

A contemplated treatment period for use in the present invention may be from about 14 to about 85 days, e.g. about 28, 50 or 70 days, in association with the insertion or repair of a stent, an indwelling shunt, fistula or catheter. The stent may remain life long in place. The polymer may disappear due to degradation.

The polymer layer coated onto the device may have a thickness in the range of from about 0.1 to 1000 $\mu$m, e.g. at least about 0.5 $\mu$m, e.g. up to 20 $\mu$m, e.g. from about 1 to 1000 $\mu$m. In one aspect of the invention, the thickness of the polymer layer may, advantageously, be used to influence the release duration of the pharmacologically active agent. The overall amount of drug released per time may be influenced by drug loading and the polymer surface.

There is further provided a use for preventing, treating, reducing or stabilizing

(i) smooth muscle cell proliferation and migration in hollow tubes, e.g. catheter-based device, or increased cell proliferation or decreased apoptosis or increased matrix deposition;

(ii) intimal thickening in vessel walls, e.g. remodeling, hypertrophic remodeling, matrix deposition, fibrin deposit, neointima growth, stenosis, restenosis, e.g. following revascularization or neovascularization, and/or inflammation and/or thrombosis;

(iii) inflammatory disorders, e.g. T-cell induced inflammation, in hollow tubes;

(iv) stabilizing vulnerable plaques in blood vessels;

(v) restenosis, e.g. in diabetic or hypertensive patients;

(vi) vascular access dysfunction, e.g. in dialysis, e.g. hemodialysis, patients,

(vii) arterial or venous aneurisms;

(viii) anastomic hyperplasia;

(ix) arterial, e.g. aortic, by-pass anastomosis;

(x) infectious diseases;

in a subject in need thereof which comprises the use, e.g. insertion or repair, of a device, e.g. any catheter-based device, e.g. indwelling shunt, fistula or catheter, e.g. a large bore catheter, intraluminal medical device, or adventitial medical device, e.g. into a vein or artery, wherein the device is coated with the polymer as hereinabove described, e.g. in conjunction with one or more pharmacologically active ingredients, e.g. as hereinabove described.

In a further aspect the invention provides a drug delivery device or system comprising a medical device adapted for local application or administration in hollow tubes, e.g. a catheter-based delivery device, e.g. an indwelling shunt, fistula or catheter, or a medical device intraluminal or outside of hollow tubes such as an implant or a sheath placed within the adventitia, coated with the polymer as described herein, and a therapeutic dosage of a pharmacologically active agent incorporated into the polymer.

Such a local delivery device or system can be used to reduce the herein mentioned vascular injuries e.g. stenosis, restenosis, or in-stent restenosis, as an adjunct to revascularization, bypass or grafting procedures performed in any vascular location including coronary arteries, carotid arteries, renal arteries, peripheral arteries, cerebral arteries or any other arterial or venous location, to reduce anastomic stenosis or hyperplasia, including in the case of arterial-venous dialysis access, or in conjunction with any other heart or transplantation procedures, or congenital vascular interventions.

In yet a further aspect the invention provides a device coated with a polymer as defined hereinabove

The invention further provides the use of a a biodegradable polymer, comprising ethylene carbonate units of the formula -(-C(O)-O-CH$_2$-CH$_2$-O-)- having an ethylene carbonate content of 70 to 100 Mol%, an intrinsic viscosity of 0.4 to 4.0 dl/g measured in chloroform at 20°C at a concentration of 1 g/dl and a glass transition temperature of from 5 to 50°C, e.g. 15 to 50°C, degradable by surface erosion which is governed by a non-hydrolytic mechanism, optionally in conjunction with a pharmacologically active agent, for the coating of a device, e.g. a medical device, e.g. a stent, e.g. for use in any method as defined under (i) to (x).

For example, the pharmacologically active agent(s) may be incorporated into the polymer or polymeric matrix of the invention, e.g. dissolved, dispersed or suspended in a solution of the polymer, and sprayed onto the outer surface of the stent. A mixture of the drug(s) and the polymeric material may be prepared in a solvent or a mixture of solvents and applied to the surfaces of the stents also by dip-coating, brush coating, e.g. airbrush coating, printing and/or dip/spin coating, the solvent (s) being allowed to evaporate to leave a film with entrapped drug(s). As solvents for example dichloromethane or methylene chloride may be used.

EP 1 699 503 B1

A process for the production of the device wherein the device is pre-covered with a polymer, e.g. a biocompatible and/or non-biodegradable polymer, and then covered with the polymer containing the drug dissolved, dispersed or suspended therein is described.

Coating thickness may depend on factors such as viscosity, e.g. polymer concentration, solvent nature, spray rate, e.g. as known to one skilled in the art. In order to increase the coating thickness and with this the drug release duration, additional layers may be sprayed onto the already coated stent. Alternatively, the spray rate and drying rate may be adjusted in such a way that a continuous coating process results.

Utility of the device of the invention in treating, preventing, promoting or stabilizing conditions as hereinabove described, may be demonstrated in animal tests or standard clinical trials, for example using dosages of pharmacologically active agents within the range of 0.001 - 100 mg/kg, e.g. 0.001 - 10 mg/kg, body weights. The effect of the device of the invention in treating or preventing neointimal proliferation and thickening, restenosis and/or vascular occlusion following vascular injury or for promoting tissue healing can be monitored by any of the methods known to one skilled in the art, e.g. reduction in the extent of restenotic lesion formation compared with placebo treatment, for example reduction in average neointimal thickness, neointimal area reduction, and percent arterial stenosis reduction, (neo)intimal and endothelial healing, suppression of in-stent neointimal growth and remodeling, e.g. hypertrophic remodeling, reduction in fibrin deposit.

One animal test may be affected as follows:

A combined angioplasty and stenting procedure is performed in New Zealand White rabbit iliac arteries. Iliac artery balloon injury is performed by inflating a 3.0 x 9.0 mm angioplasty balloon in the mid-portion of the artery followed by "pull-back" of the catheter for 1 balloon length. Balloon injury is repeated 2 times, and a 3.0 x 12 mm stent coated according to the invention is deployed at 6 atm for 30 seconds in the iliac artery. Balloon injury and stent placement is then performed on the contralateral iliac artery in the same manner. A post-stent deployment angiogram is performed. All animals receive oral aspirin 40 mg/day daily as anti-platelet therapy and are fed standard low-cholesterol rabbit chow. Twenty-eight days after stenting, animals are anesthetized and euthanized and the arterial tree is perfused at 100 mmHg with lactated Ringer's for several minutes, then perfused with 10% formalin at 100 mmHg for 15 minutes. The vascular section between the distal aorta and the proximal femoral arteries is excised and cleaned of periadventitial tissue. The stented section of artery is embedded in plastic and sections are taken from the proximal, middle, and distal portions of each stent. All sections are stained with hematoxylin-eosin and Movat pentachrome stains. Computerized planimetry is performed to determine the area of the internal elastic lamina (IEL), external elastic lamina (EEL) and lumen. The neointima and neointimal thickness is measured both at and between the stent struts. The vessel area is measured as the area within the EEL. Data are expressed as mean $\pm$ SEM. Statistical analysis of the histologic data is accomplished using analysis of variance (ANOVA) due to the fact that two stented arteries are measured per animal with a mean generated per animal. A $P < 0.05$ is considered statistically significant.

Preferred pharmacologically active agents or combinations of pharmacologically active agents for use in the animal tests or standard clinical trials are those having antiproliferative properties, e.g. taxol, paclitaxel, docetaxel, an epothilone, a tyrosine kinase inhibitor, a VEGF receptor tyrosine kinase inhibitor, a VEGF receptor inhibitor, a compound binding to VEGF, a mTOR inhibitor agent e.g. rapamycin derivatives, e.g. 40-O-(2-hydroxyethyl)-rapamycin, a compound having anti-inflammatory properties, e.g. a steroid, a cyclooxygenase inhibitor.

The polymer used in the device of the invention is biodegradable and shows superior release, tolerability, biocompatibility and mechanical properties. For example, the ethylene carbonate polymer used herein is extremely viscoelastic, for example can be stretched up to 1000%, e.g. 500-1000%, without rupture, depending also on the polymer molecular weight. The drug release from the device, e.g. stent, can be controlled by the coating composition, e.g. the process for manufacturing the polymer, the amount and/or the particle size of the drug, as well as the amount of superoxid radicals present during a restenosis process. Due to the degradation of the polymer by surface erosion which is governed by a non-hydrolytic mechanism, the active compound is protected by the polymer. The active compound will be released during the degradation process and will be completely protected from the blood environment until the polymer erodes. Due to the biocompatibility of the polymer, no or only minor inflammatory reactions occur. Accordingly, the device of the invention shows improved long-term success of the procedure employing the device, e.g. stenting procedure. Preferably the smooth muscle cell proliferation or migration is inhibited or reduced according to the invention immediately proximal or distal to the locally treated or stented area.

Without further elaboration, it is believed that one skilled in the art can, using the preceding description, utilize the present invention to its fullest extent. The following example is, therefore, to be construed as merely illustrative and not a limitation of the scope of the present invention in any way.

In the following examples 40-O-(2-hydroxyethyl)-rapamycin (RAD), e.g. in a concentration of 0.05 to 25 mg/ml, is used as a drug (hereinafter "Compound I") for incorporation into the polymer of the invention. Other drugs, e.g. as mentioned hereinabove, may be suspended in the polymer solution.

Example 1: A stent is manufactured from medical 316LS stainless steel and is composed of a series of cylindrically oriented rings aligned along a common longitudinal axis. Each ring consists of 3 connecting bars and 6 expanding elements. The stent is premounted on a delivery system.

1.1 Compound I, optionally together with 2,6-di-tert.-butyl-4-methylphenol (0.001 mg/ml), is incorporated into a polymer matrix based on a polymer which comprises ethylene carbonate units of the formula -(-C(O)-O-CH$_2$CH$_2$-O-)- having an ethylene carbonate content of 70 to 100 Mol%, an intrinsic viscosity of 0.4 to 4.0 dl/g measured in chloroform at 20°C at a concentration of 1 g/dl and a glass transition temperature of from 15 to 50°C, degradable by surface erosion which is governed by a non-hydrolytic mechanism (the polymer of the invention, PEC). The stent is coated with this matrix.

1.2 1 g PEC and 50 mg Compound I are dissolved in 10 ml methylene chloride. This solution is sprayed onto the stent. After drying using a defined gas flow or vacuum, a defined polymer/drug film remains on the stent.

Other methods to coat the stent may be used, e.g. dipping, brushing, printing or spin coating.

Example 2A:

PEC is synthesized according to the procedure hereinabove described. Stents are airbrush coated with a solution of PEC or Poly (D,L-lactic-co-glycolic acid) (PLGA, RG502H Resomer 50:50), from Boehringer Ingelheim, (Ingelheim, Germany), in dichloromethane forming a polymer matrix covering the outer, vessel wall-directed surface of the stent.

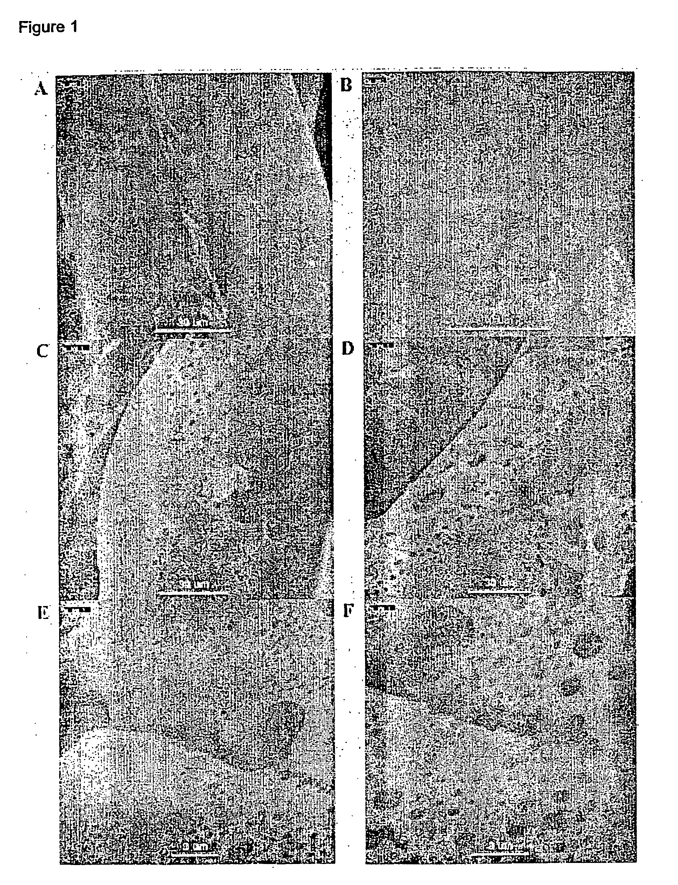

PEC and PLGA coated stents are expanded using an enclosed balloon catheter and coating condition after expansion is examined by scanning electron miscroscopy (SEM) using a Hitachi S-41 00 microscope (Hitachi, Germany). The flexibly PEC coated stent shows a surface without any signs of disintegration (Fig. 1 (A, B)), while the PLGA coated stent shows ruptures and cracks at highly burdened stent parts (Fig. 1 (C-F).

These ruptures and cracks may induce fast restenosis.

Example 2B:

Stents are completely pre-covered with poly-p-xylylene (PPX) using the chemical vapour deposition method as described in e.g. Gorham WF, J. Polym. Sci. Polym. Chem. 1966; 4(12):3027-39, before they are coated with a solution of PEC or PLGA.

Example 2C:

Compound I is incorporated into the solution of PEC or PLGA before forming a polymer matrix covering the outer surface of the stents.

Figure 1:

Scanning electron micrographs of airbrush - coated stents after balloon dilatation.
Smooth PEC surface coating without any signs of disintegration at 1000x (A) and 4000 x magnification (dust particle as focusing aid) (B). RG502H (PLGA) surface coating showing ruptures at 900 x magnification (C) and cracks at highly burdened stent parts at 800 x (D), 6000 x (E) and 7500 x magnification (F).

**Claims**

1. A device having a surface, the surface having a coating comprising a biodegradable polymer which comprises ethylene carbonate units of the formula A

$$-(-C(0)-0-CH_2-CH_2-0-)- \qquad A$$

having an ethylene carbonate content of 70 to 100 Mol%, an intrinsic viscosity of 0.4 to 4.0 dl/g measured in chloroform at 20°C at a concentration of 1 g/dl and a glass transition temperature of from 5 to 50°C, degradable by surface erosion which is governed by a non-hydrolytic mechanism.

2. The device of claim 1 further comprising a pharmacologically active agent.

3. The device of claim 2 wherein the pharmacologically active agent is dissolved or dispersed in the polymer.

4. The device of claim 2 or 3 containing an immunosuppressant or antiproliferative agent as pharmacologically active agent.

5. The device of any preceding claim in form of a stent or catheter.

6. The device of claim 5 in form of a drug-eluting stent.

7. Use of the device of any preceding claim for the controlled release of a pharmacologically active agent.

8. Use of a biodegradable polymer, comprising ethylene carbonate units of the formula A

$$-(-C(0)-0-CH_2-CH_2-0-)- \qquad A$$

having an ethylene carbonate content of 70 to 100 Mol%, an intrinsic viscosity of 0.4 to 4.0 dl/g measured in chloroform at 20°C at a concentration of 1 g/dl and a glass transition temperature of from 5 to 50°C, degradable by surface erosion which is governed by a non-hydrolytic mechanism for the coating of a device.

**Patentansprüche**

1. Vorrichtung mit einer Oberfläche, wobei die Oberfläche eine Beschichtung aufweist, die ein biologisch abbaubares Polymer, das Ethylencarbonateinheiten der Formel A

$$-(-C(O)-O-CH_2-CH_2-O-)- \qquad A$$

umfasst, mit einem Ethylencarbonatgehalt von 70 bis 100 Mol%, einer intrinsischen Viskosität von 0,4 bis 4,0 dl/g, gemessen in Chloroform bei 20°C bei einer Konzentration von 1 g/dl, und einer Glasübergangstemperatur von 5 bis 50°C umfasst, das durch Oberflächenerosion, die durch einen nichthydrolytischen Mechanismus gesteuert wird, abbaubar ist.

2. Vorrichtung nach Anspruch 1, die des Weiteren ein pharmakologisch aktives Mittel umfasst.

3. Vorrichtung nach Anspruch 2, wobei das pharmakologisch aktive Mittel in dem Polymer gelöst oder dispergiert ist.

4. Vorrichtung nach Anspruch 2 oder 3, die ein immunosuppressives oder antiproliferatives Mittel als pharmakologisch aktives Mittel enthält.

5. Vorrichtung nach einem der vorhergehenden Ansprüche in Form eines Stents oder Katheters.

6. Vorrichtung nach Anspruch 5 in Form eines Arzneimittel abgebenden Stents.

7. Verwendung der Vorrichtung nach einem der vorhergehenden Ansprüche zur gesteuerten Freisetzung eines pharmakologisch aktiven Mittels.

8. Verwendung eines biologisch abbaubaren Polymers, das Ethylencarbonateinheiten der Formel A umfasst

$$-(-C(O)-O-CH_2-CH_2-O-)- \qquad A,$$

mit einem Ethylencarbonatgehalt von 70 bis 100 Mol-%, einer intrinsischen Viskosität von 0,4 bis 4,0 dl/g, gemessen in Chloroform bei 20°C bei einer Konzentration von 1 g/dl, und einer Glasübergangstemperatur von 5 bis 50°C, das durch Oberflächenerosion abbaubar ist, die durch einen nichthydrolytischen Mechanismus gesteuert wird, zum Beschichten einer Vorrichtung.

**Revendications**

1.  Dispositif ayant une surface, la surface ayant un revêtement comprenant un polymère biodégradable qui comprend des motifs carbonate d'éthylène de formule A

    $$-(-C(O)-O-CH_2-CH_2-O-)-\qquad A$$

    ayant une teneur en carbonate d'éthylène de 70 à 100 % en moles, une viscosité intrinsèque de 0,4 à 4,0 dl/g, mesurée dans le chloroforme à 20°C à une concentration de 1 g/dl et une température de transition vitreuse de 5 à 50°C, dégradable par une érosion superficielle qui est pilotée par un mécanisme non hydrolytique.

2.  Dispositif selon la revendication 1, qui comprend en outre un principe pharmacologiquement actif.

3.  Dispositif selon la revendication 2, dans lequel le principe pharmacologiquement actif est dissous ou dispersé dans le polymère.

4.  Dispositif selon la revendication 2 ou 3, contenant un immunosuppresseur ou un agent antiprolifératif en tant que principe pharmacologiquement actif.

5.  Dispositif selon l'une quelconque des revendications précédentes, sous forme d'un stent ou d'un cathéter.

6.  Dispositif selon la revendication 5, sous forme d'un stent à élution de médicament.

7.  Utilisation du dispositif selon l'une quelconque des revendications précédentes pour la libération contrôlée d'un principe pharmacologiquement actif.

8.  Utilisation, pour le revêtement d'un dispositif, d'un polymère biodégradable comprenant des motifs carbonate d'éthylène de formule A

    $$-(-C(O)-O-CH_2-CH_2-O-)-\qquad A$$

    ayant une teneur en carbonate d'éthylène de 70 à 100 % en moles, une viscosité intrinsèque de 0,4 à 4,0 dl/g, mesurée dans le chloroforme à 20°C à une concentration de 1 g/dl et une température de transition vitreuse de 5 à 50°C, dégradable par une érosion superficielle qui est pilotée par un mécanisme non hydrolytique.

Figure 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9506077 A
- WO 9606068 A
- WO 9845249 A
- EP 0296110 A
- US 5093330 A
- JP 2708047 B
- WO 9702266 A
- EP O564409 A
- WO 9903854 A
- EP 0520722 A
- EP 0566226 A
- EP 0787722 A
- EP 0837063 A
- US 5747498 A
- WO 9810767 A
- WO 9730034 A
- WO 9749688 A
- WO 9738983 A
- WO 9630347 A
- WO 9633980 A
- WO 9503283 A
- WO 9835958 A
- WO 0009495 A
- WO 0027820 A
- WO 0059509 A
- WO 9811223 A
- WO 0027819 A
- WO 0037502 A
- WO 9410202 A
- EP 0769947 A
- WO 9409010 A
- WO 9641807 A
- WO 9802441 A
- WO 0114387 A

**Non-patent literature cited in the description**

- **MEYER T ; REGENASS U ; FABBRO D et al.** *Int J Cancer,* 1989, vol. 43, 851-856
- **M. PREWETT et al.** *Cancer Research,* 1999, vol. 59, 5209-5218
- **F. YUAN et al.** *Proc. Natl. Acad. Sci. USA,* December 1996, vol. 93, 14765-14770
- **Z. ZHU et al.** *Cancer Res.,* 1998, vol. 58, 3209-3214
- **J. MORDENTI et al.** *Toxicologic Pathology,* 1999, vol. 27 (1), 14-21
- **M. S. O'REILLY et al.** *Cell,* 1994, vol. 79, 315-328
- **M. S. O'REILLY et al.** *Cell,* 1997, vol. 88, 277-285
- **GORHAM WF.** *J. Polym. Sci. Polym. Chem.,* 1966, vol. 4 (12), 3027-39